# EUROPÄISCHE PATENTSCHRIFT

(11) **EP 3 658 063 B1**
(45) Veröffentlichungstag und Bekanntmachung des Hinweises auf die Patenterteilung: **27.12.2023**
(21) Anmeldenummer: 18746670.1
(22) Anmeldetag: 25.07.2018
(51) Int. Cl.: A61B 90/98, A61B 17/32

(54) **VERFAHREN ZUM INTEGRIEREN EINER ELEKTRISCHEN SCHALTUNG IN EINE VORRICHTUNG UND VORRICHTUNG**
METHOD FOR INTEGRATING AN ELECTRICAL CIRCUIT IN A DEVICE AND DEVICE
PROCÉDÉ POUR INTÉGRER UN CIRCUIT ÉLECTRIQUE DANS UN DISPOSITIF ET DISPOSITIF

(30) Priorität: 28.07.2017 DE 102017213080
(43) Veröffentlichungstag der Anmeldung: 03.06.2020
(73) Patentinhaber: Robert Bosch GmbH, 70442 Stuttgart (DE)
(72) Erfinder: BLAESIUS, Jana, 71638 Ludwigsburg (DE); ECHANIZ, Aitor, 70197 Stuttgart (DE); SCHOEPF, Martin, 70499 Stuttgart (DE); WALTHER, Michael, 71272 Renningen (DE)
(86) Internationale Anmeldenummer: PCT/EP2018/070176
(87) Internationale Veröffentlichungsnummer: WO 2019/020693

(56) Entgegenhaltungen:
- WO-A1-2014/017530
- WO-A2-2011/054355
- US-A1- 2005 223 550
- US-A1- 2010 176 925

## Beschreibung

### Stand der Technik

Die Erfindung geht von einer Vorrichtung oder einem Verfahren nach Gattung der unabhängigen Ansprüche aus

Eine Integration von Sensorik, wie z. B. RFID-Chips (RFID = Radio-Frequency Identification; Identifizierung mit Hilfe elektromagnetischer Wellen), kann unter anderem beispielsweise im Bereich von Wartung und Instandhaltung und im Bereich Logistik zur Anwendung kommen häufig kann. Häufig kann dabei ein RFID-Transponder an bestimmten Stellen und Werkzeugen temporär befestigt, z. B. geklebt, werden.

Die US 6,972,199 B2 bezieht sich auf ein Schneidinstrument, welches eine Vielzahl von Sensoren aufweist, die darin integriert sind, bzw. auf in Halbleitermaterial eingebettete Sensorik.

Die WO 11/054355 A2 offenbart ein Verfahren zum Aufbringen beispielsweise eines RFID-Chips auf beispielsweise einer Oberfläche eines medizinischen Instruments unter Aufbringung einer Schutzschicht.

Die US 2005/0223550 A1 offenbart ein Verfahren um ein Mikroschaltungsmodul, welches einen Mikroschaltkreis trägt, unlösbar in einem Kartenkörper zu montieren.

### Offenbarung der Erfindung

Vor diesem Hintergrund werden mit dem hier vorgestellten Ansatz ein Verfahren und eine Vorrichtung gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Gemäß Ausführungsformen kann insbesondere ein Werkzeug oder eine andere Vorrichtung als ein Verbundteil aus zwei Werkstoffen mit integrierter elektrischer Schaltung realisiert werden, wobei im Grenzflächenbereich zwischen den zwei Werkstoffen aus einem ersten der Werkstoffe eine Mehrzahl von Verbindungselementen zur Erhöhung einer Adhäsion zwischen den Werkstoffen ausgeformt sind. Durch eine neuartige Auslegung von Werkzeugen, Operationsbesteck oder anderen Vorrichtungen als Verbundteile, beispielsweise als Metall-Kunststoff-Verbundteile, kann eine Integration von elektrischen Schaltungen, insbesondere Sensorik, vorteilhaft realisiert werden.

Somit kann eine feste und dichte Verbindung zwischen den Werkstoffen, beispielsweise einem Metallwerkzeug und einem aufgebrachten Kunststoffvolumen, in dem die elektrische Schaltung eingebettet sein kann, erreicht werden. Zur Realisierung einer zuverlässigen Verbindungsstelle zwischen den Werkstoffen können Verbindungselemente, beispielsweise in Gestalt von Aussparungen, in einem den ersten Werkstoff aufweisenden Abschnitt der Vorrichtung vorgesehen werden, welche beispielsweise während des Aufbringens des zweiten Werkstoffs, beispielsweise Spritzgießens von Kunststoff, durch den zweiten Werkstoff umflossen werden können und somit eine robuste Umklammerung bzw. Verankerung des zweiten Werkstoffs an den Verbindungselementen ermöglichen. Es kann somit ein Werkzeug oder eine andere Vorrichtung als ein Verbundteil, insbesondere ein Kunststoff-Metall-Verbundteil, realisiert werden, um eine haltbare, dauerhafte und sterilisationssichere Möglichkeit zur Integration von elektrischen Schaltungen beispielsweise in metallische Werkzeuge zu ermöglichen.

Es wird ein Verfahren zum Integrieren einer elektrischen Schaltung in eine Vorrichtung vorgestellt, wobei die Vorrichtung eine Integrationsoberfläche aus einem ersten Werkstoff aufweist, und wobei das Verfahren zumindest folgende Schritte aufweist:
Bearbeiten der Integrationsoberfläche, um Verbindungselemente zum Erhöhen einer Adhäsion eines zweiten Werkstoffs an der Integrationsoberfläche auszuformen, wobei sich der zweite Werkstoff von dem ersten Werkstoff unterscheidet
Anordnen der elektrischen Schaltung benachbart zu der bearbeiteten Integrationsoberfläche; und
Aufbringen eines Volumens des zweiten Werkstoffs zumindest über die Integrationsoberfläche, um die elektrische Schaltung fluiddicht einzuhausen.

Die Vorrichtung kann ein Werkzeug, ein medizintechnisches Werkzeug, ein Operationsbesteck oder dergleichen sein. Die elektrische Schaltung kann auf einer Leiterplatte oder dergleichen realisiert sein. Die elektrische Schaltung kann zusätzlich oder alternativ elektronische Bauteile oder Komponenten aufweisen. Der erste Werkstoff kann ein metallischer Werkstoff sein. Der zweite Werkstoff kann ein Kunststoff sein. Im Schritt des Bearbeitens kann ein Oberflächenmaterial der Integrationsoberfläche verformt werden, um die Verbindungselemente auszuformen. Die Integrationsoberfläche kann einen Teilabschnitt einer Oberfläche der Vorrichtung umfassen, der zur Integration der elektrischen Schaltung vorgesehen ist. Dabei kann die Integrationsoberfläche in einem den ersten Werkstoff aufweisenden Abschnitt oder Teilabschnitt der Vorrichtung angeordnet sein. Die Integrationsoberfläche kann eine Schicht, beispielsweise eine Grenzschicht, des ersten Werkstoffs darstellen. Im Schritt des Anordnens kann die elektrische Schaltung direkt auf die Integrationsoberfläche aufgebracht werden und im direkten Kontakt mit der Integrationsoberfläche stehen. Benachbart kann direkt angrenzend oder mit einem Luftspalt oder fertigungsbedingten Zwischenraum indirekt angrenzend repräsentieren. Im Schritt des Anordnens kann die elektrische Schaltung auf oder über einem Bereich der Integrationsoberfläche aufgebracht werden. Im Schritt des Aufbringens kann der zweite Werkstoff auf die elektrische Schaltung aufgebracht werden. Vorteilhafterweise können sich die Verbindungselemente positiv in Bezug auf eine elektromagnetische Abschirmung der elektrischen Schaltung auswirken. Zudem können sich die Verbindungselemente positiv auf die Stabilität der Verbindung zwischen dem ersten und den zweiten Werkstoff auswirken. Optional kann mindestens ein Teilabschnitt des den ersten Werkstoff aufweisenden Teils der Vorrichtung mittels eines additiven Fertigungsprozesses ausgeformt sein oder werden.

Gemäß einer Ausführungsform können im Schritt des Bearbeitens Gitterelemente, Ausnehmungen, Schlaufen, Vorsprungsabschnitte, Winkelelemente, Kavitäten mit Hinterschnitten und zusätzlich oder alternativ Kavitäten zur Erhöhung eines Rauigkeitswertes als Verbindungselemente ausgeformt werden. Eine solche Ausführungsform bietet den Vorteil, dass mit unaufwendig und konstruktiv einfach zu realisierenden Mitteln eine zuverlässige und robuste Verbindung zwischen den unterschiedlichen Werkstoffen erreicht werden kann. Eine Auswahl von geeigneten Verbindungselementen kann hierbei anwendungsabhängig und Werkstoff abhängig flexibel erfolgen.

Auch können im Schritt des Bearbeitens die Verbindungselemente zusätzlich mittels Laserstrukturierens, Funkenerodierens, Umformens und zusätzlich oder alternativ Zerspanens ausgeformt werden. Eine solche Ausführungsform bietet den Vorteil, dass gut bekannte und verfügbare Bearbeitungstechniken zum genauen Ausformen der Verbindungselemente eingesetzt werden können.

Im Schritt des Bearbeitens werden mindestens die Verbindungselemente mittels eines additiven Fertigungsprozesses ausgeformt. Bei dem additiven Fertigungsprozess kann es sich beispielsweise um 3D-Druck bzw. dreidimensionalen Druck handeln. Eine solche Ausführungsform bietet den Vorteil, dass auch komplexe Strukturen genau, einfach und zuverlässig erzeugt werden können.

Ferner können im Schritt des Bearbeitens zumindest ein Teil der Verbindungselemente als zumindest eine Antenne für Signale zu und zusätzlich oder alternativ von der elektrischen Schaltung ausgeformt werden. Somit kann die Vorrichtung zumindest eine Antenne aufweisen, die durch die Verbindungselemente gebildet oder ausgeformt ist. Hierbei kann die elektrische Schaltung signalübertragungsfähig mit den Verbindungselementen verbindbar oder verbunden sein. Eine solche Ausführungsform bietet den Vorteil, dass Signale zu bzw. von der elektrischen Schaltung verstärkt und gegebenenfalls gerichtet gesendet und empfangen werden können.

Dabei kann die zumindest eine Antenne als eine Wendelantenne, als eine Wendelantenne mit radialen Mikrokavitäten, als eine Rechteckantenne und zusätzlich oder alternativ als eine Schmetterlingsantenne ausgeformt werden. Eine solche Ausführungsform bietet den Vorteil, dass ein auf die spezielle Anwendung sowie eine Frequenz der elektrischen Schaltung abgestimmter und somit geeigneter Antennentyp ausgewählt werden kann.

Hierbei können auch eine Mehrzahl von Antennen ausgeformt werden, die in unterschiedlichen Dimensionen ausgerichtet sind. Die Mehrzahl von Antennen können durch dreidimensional ausgerichtete oder dreidimensionale Verbindungselemente gebildet oder ausgeformt sein. Eine solche Ausführungsform bietet den Vorteil, dass eine zuverlässige Signalübertragung in unterschiedliche Raumrichtungen bzw. aus unterschiedlichen Raumrichtungen platzsparende und unaufwendig realisiert werden kann.

Auch kann dabei im Schritt des Bearbeitens zumindest ein Vertiefungsabschnitt in der Integrationsoberfläche ausgeformt werden, wobei der zumindest eine Vertiefungsabschnitt ein parabolisches Vertiefungsprofil aufweist. Eine solche Ausführungsform bietet den Vorteil, dass auf einfache Weise ein Parabolspiegel zur Verbesserung eines Verhaltens der zumindest einen Antenne geschaffen werden kann. Somit kann ein gerichtetes Abstrahlen von Signalen zuverlässig erreicht werden.

Ferner kann im Schritt des Anordnens eine elektrische Schaltung angeordnet werden, die zumindest eine Erfassungseinrichtung zum Erfassen einer physikalischen Größe und zusätzlich oder alternativ eine Signalübertragungseinrichtung zum Übertragen von Signalen zu und zusätzlich oder alternativ von der elektrischen Schaltung aufweisen kann. Die physikalische Größe kann eine Temperatur, eine Beschleunigung oder dergleichen repräsentieren. Eine solche Ausführungsform bietet den Vorteil, dass eine Positionsbestimmung bzw. ein Auffinden der Vorrichtung und zusätzlich oder alternativ ein Nachweis einer speziellen Behandlung der Vorrichtung, beispielsweise durch Einwirkung einer Temperatur, ermöglicht bzw. erleichtert werden kann.

Gemäß einer Ausführungsform kann im Schritt des Anordnens die elektrische Schaltung mit einem Kapselungsmaterial verkapselt werden und/oder mit einer Wärmeschutzschicht versehen werden. Eine solche Ausführungsform bietet den Vorteil, dass die elektrische Schaltung somit im Hinblick auf den Schritt des Aufbringens vor einer Einwirkung des zweiten Werkstoffs und zusätzlich oder alternativ einer Temperatur geschützt werden kann. Ferner können eine Lebensdauer verlängert sowie ein thermischer Einsatzbereich der elektrischen Schaltung vergrößert werden.

Es wird auch eine Vorrichtung vorgestellt, die zumindest folgende Merkmale aufweist:
eine Integrationsoberfläche aus einem ersten Werkstoff, wobei an der Integrationsoberfläche Verbindungselemente zum Erhöhen einer Adhäsion eines zweiten Werkstoffs an der Integrationsoberfläche ausgeformt sind, wobei sich der zweite Werkstoff von dem ersten Werkstoff unterscheidet;
eine benachbart zu der bearbeiteten Integrationsoberfläche angeordnete elektrische Schaltung; und
ein zumindest über die Integrationsoberfläche aufgebrachtes Volumen des zweiten Werkstoffs zum fluiddichten Einhausen der elektrischen Schaltung,
wobei bei dem Schritt des Bearbeitens die Verbindungselemente mittels eines additiven Fertigungsprozesses ausgeformt werden.

Die elektrische Schaltung kann durch Ausführen von Schritten einer Ausführungsform des vorstehend genannten Verfahrens in die Vorrichtung integriert sein.

Beispielsweise kann die Vorrichtung als ein medizinisches Gerät ausgeformt sein und die Integrationsoberfläche kann an einem Griff des medizinischen Geräts ausgeformt sein.

Ausführungsbeispiele des hier vorgestellten Ansatzes sind in den Zeichnungen dargestellt und in der nachfolgenden Beschreibung näher erläutert. Es zeigt:
Fig. 1 eine schematische Darstellung einer Vorrichtung gemäß einem Ausführungsbeispiel;
Fig. 2 ein Ablaufdiagramm eines Verfahrens zum Integrieren gemäß einem Ausführungsbeispiel;
Fig. 3 eine schematische Darstellung einer Vorrichtung gemäß einem Ausführungsbeispiel;
Fig. 4 eine schematische Darstellung einer Vorrichtung gemäß einem Ausführungsbeispiel;
Fig. 5 eine schematische Darstellung von Verbindungselementen gemäß einem Ausführungsbeispiel;
Fig. 6 eine schematische Darstellung von Verbindungselementen gemäß einem Ausführungsbeispiel;
Fig. 7 eine schematische Darstellung von Verbindungselementen gemäß einem Ausführungsbeispiel;
Fig. 8 eine schematische Darstellung von Verbindungselementen gemäß einem Ausführungsbeispiel;
Fig. 9 eine schematische Darstellung von Verbindungselementen gemäß einem Ausführungsbeispiel;
Fig. 10 eine schematische Darstellung von Verbindungselementen gemäß einem Ausführungsbeispiel;
Fig. 11 eine schematische Darstellung von Verbindungselementen gemäß einem Ausführungsbeispiel;
Fig. 12 eine schematische Darstellung von Verbindungselementen gemäß einem Ausführungsbeispiel;
Fig. 13 eine schematische Darstellung von Verbindungselementen gemäß einem Ausführungsbeispiel;
Fig. 14 eine schematische Darstellung von Verbindungselementen gemäß einem Ausführungsbeispiel; und
Fig. 15 eine schematische Darstellung von Verbindungselementen gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

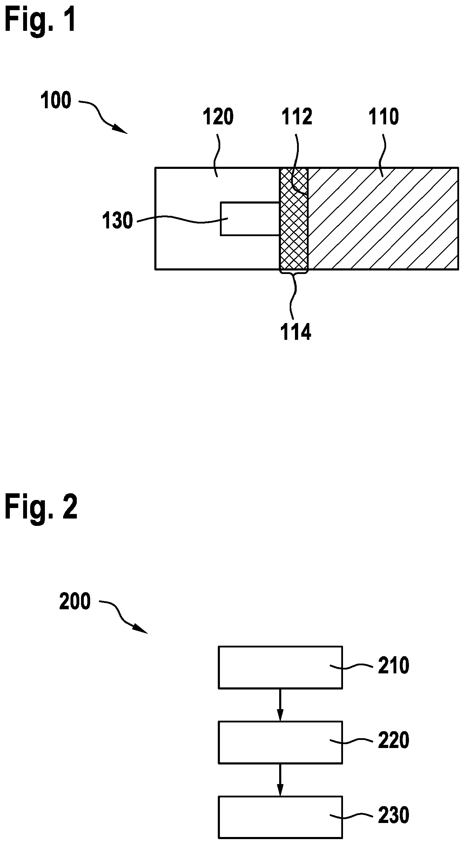

Fig. 1 zeigt eine schematische Darstellung einer Vorrichtung 100 gemäß einem Ausführungsbeispiel. Bei der Vorrichtung 100 handelt es sich um ein Werkzeug, beispielsweise um ein medizinisches Gerät, wie beispielsweise Operationsbesteck oder dergleichen, oder eine andere Art von Vorrichtung.

Die Vorrichtung 100 weist ein Volumen eines ersten Werkstoffs 110 auf. Bei dem ersten Werkstoff 110 handelt es sich beispielsweise um ein Metallmaterial. Das Volumen des ersten Werkstoffs 110 weist eine Integrationsoberfläche 112 auf. Somit ist die Integrationsoberfläche 112 aus dem ersten Werkstoff 110 ausgeformt. Ferner sind an der Integrationsoberfläche 112 eine Mehrzahl von Verbindungselemente 114 durch Bearbeiten der Integrationsoberfläche 112 bzw. des ersten Werkstoffs 110 ausgeformt.

Auch weist die Vorrichtung 100 ein Volumen eines zweiten Werkstoffs 120 auf. Der zweite Werkstoff 120 unterscheidet sich von dem ersten Werkstoff 110. Bei dem zweiten Werkstoff 120 handelt es sich beispielsweise um ein Kunststoffmaterial. Das Volumen des zweiten Werkstoffs 120 grenzt an der Integrationsoberfläche 112 und den Verbindungselementen 114 an das Volumen des ersten Werkstoffs 110 an. Die Verbindungselemente 114 sind ausgeformt, um eine Adhäsion eines zweiten Werkstoffs 120 an der Integrationsoberfläche 112 bzw. an dem ersten Werkstoff 110 zu erhöhen.

Die Vorrichtung 100 weist ferner eine elektrische Schaltung 130 auf. Die elektrische Schaltung 130 ist benachbart zu der Integrationsoberfläche 112 angeordnet. Das Volumen des zweiten Werkstoffs 120 ist zumindest über die elektrische Schaltung 130 und die Integrationsoberfläche 112 aufgebracht. Mittels des Volumens des zweiten Werkstoffs 120 ist die elektrische Schaltung 130 fluiddicht eingehaust.

Gemäß einem Ausführungsbeispiel ist die elektrische Schaltung 130 in Gestalt eines Funkchips oder dergleichen ausgeführt. Die elektrische Schaltung 130 kann beispielsweise eine Erfassungseinrichtung zum Erfassen einer physikalischen Größe und/oder eine Signalübertragungseinrichtung zum Übertragen von Signalen aufweisen.

Die Verbindungselemente 114 sind gemäß dem in Fig. 1 dargestellten Ausführungsbeispiel als Gitterelemente ausgeformt. Zusätzlich oder alternativ sind Verbindungselemente 114 gemäß einem Ausführungsbeispiel als zumindest eine Antenne für Signale zu und/oder von der elektrischen Schaltung ausgeformt. Gemäß anderen Ausführungsbeispielen können die Verbindungselemente 114 zusätzlich oder alternativ als Ausnehmungen, Schlaufen, Vorsprungsabschnitte, Winkelelemente, Kavitäten mit Hinterschnitten und/oder Kavitäten zur Erhöhung eines Rauigkeitswertes ausgeformt sein.

Eine Höhe der Verbindungselemente 114 kann gemäß unterschiedlicher Ausführungsbeispiel im Bereich von Nanometern, Mikrometern oder Millimetern liegen.

Fig. 2 zeigt ein Ablaufdiagramm eines Verfahrens 200 zum Integrieren gemäß einem Ausführungsbeispiel. Das Verfahren 200 zum Integrieren ist ausführbar, um eine elektrische Schaltung in eine Vorrichtung zu integrieren. Dabei ist das Verfahren 200 zum Integrieren ausführbar, um die Vorrichtung aus Fig. 1 herzustellen bzw. zu fertigen, indem die elektrische Schaltung in die Vorrichtung 100 integriert wird. Anders ausgedrückt ist das Verfahren 200 zum Integrieren in Verbindung mit einer Vorrichtung ausführbar, die eine Integrationsoberfläche aus einem ersten Werkstoff aufweist.

In einem Schritt 210 des Bearbeitens wird bei dem Verfahren 200 zum Integrieren die Integrationsoberfläche bearbeitet, um Verbindungselemente zum Erhöhen einer Adhäsion eines zweiten Werkstoffs an der Integrationsoberfläche auszuformen. Der zweite Werkstoff unterscheidet sich von dem ersten Werkstoff.

Nachfolgend wird in einem Schritt 220 des Anordnens die elektrische Schaltung benachbart zu der im Schritt 210 des Bearbeitens bearbeiteten Integrationsoberfläche angeordnet. In einem Schritt 230 des Aufbringens wird ein Volumen des zweiten Werkstoffs zumindest über die Integrationsoberfläche aufgebracht, um die elektrische Schaltung fluiddicht einzuhausen.

Beispielsweise werden im Schritt 210 des Bearbeitens Gitterelemente, Ausnehmungen, Schlaufen, Vorsprungsabschnitte, Winkelelemente, Kavitäten mit Hinterschnitten und/oder Kavitäten zur Erhöhung eines Rauigkeitswertes als die Verbindungselemente ausgeformt. Dabei werden im Schritt 210 des Bearbeitens die Verbindungselemente beispielsweise zusätzlich mittels Laserstrukturierens, Funkenerodierens, Umformens und/oder Zerspanens ausgeformt. Im Schritt 210 des Bearbeitens werden mindestens die Verbindungselemente mittels eines additiven Fertigungsprozesses ausgeformt, beispielsweise mittels 3D-Druckens bzw. dreidimensionalen Druckens.

Gemäß einem Ausführungsbeispiel werden im Schritt 210 des Bearbeitens die Verbindungselemente als zumindest eine Antenne für Signale zu und/oder von der elektrischen Schaltung ausgeformt. Insbesondere wird dabei die zumindest eine Antenne als eine Wendelantenne, als eine Wendelantenne mit radialen Mikrokavitäten, als eine Rechteckantenne und/oder als eine Schmetterlingsantenne ausgeformt. Zusätzlich oder alternativ werden dabei eine Mehrzahl von Antennen ausgeformt, die in unterschiedlichen Dimensionen ausgerichtet sind. Gemäß einem weiteren Ausführungsbeispiel wird im Schritt 210 des Bearbeitens zumindest ein Vertiefungsabschnitt in der Integrationsoberfläche ausgeformt. Der zumindest eine Vertiefungsabschnitt weist dabei insbesondere ein parabolisches Vertiefungsprofil auf.

Gemäß einem Ausführungsbeispiel wird im Schritt 220 des Anordnens eine elektrische Schaltung angeordnet, die zumindest eine Erfassungseinrichtung zum Erfassen einer physikalischen Größe und/oder eine Signalübertragungseinrichtung zum Übertragen von Signalen zu und/oder von der elektrischen Schaltung aufweist. Insbesondere wird im Schritt 220 des Anordnens eine elektrische Schaltung angeordnet, die als ein Funkchip und/oder Sensorchip ausgeführt ist. Optional wird die elektrische Schaltung im Schritt 220 des Anordnens mit einem Kapselungsmaterial verkapselt und/oder mit einer Wärmeschutzschicht versehen.

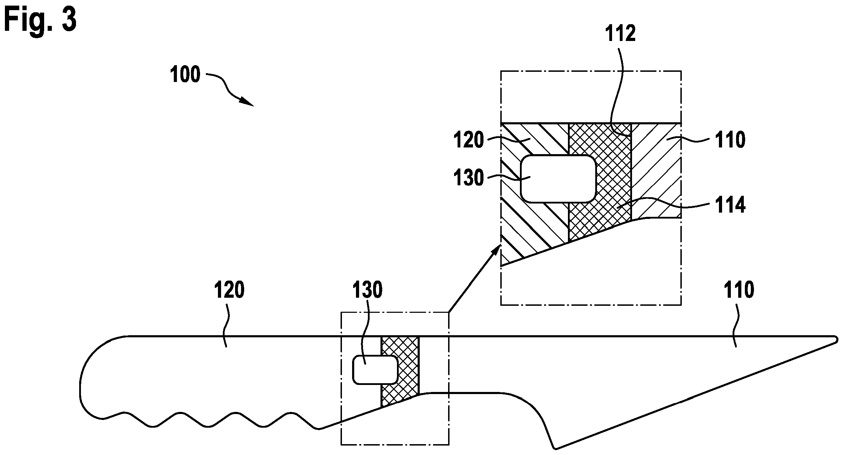

Fig. 3 zeigt eine schematische Darstellung einer Vorrichtung 100 gemäß einem Ausführungsbeispiel. Die Vorrichtung 100 in Fig. 3 entspricht oder ähnelt hierbei der Vorrichtung aus Fig. 1. Gemäß dem in Fig. 3 dargestellten Ausführungsbeispiel ist die Vorrichtung 100 als ein Operationsbesteck, insbesondere ein Skalpell oder dergleichen, ausgeführt. Hierbei repräsentiert ein eine Klinge aufweisender Teilabschnitt der Vorrichtung 100 das Volumen des ersten Werkstoffs 110. Bei dem ersten Werkstoff 110 handelt es sich um ein Metallmaterial. Ferner repräsentiert ein einen Handgriff aufweisender Teilabschnitt der Vorrichtung 100 das Volumen des zweiten Werkstoffs 120. Bei dem zweiten Werkstoff 120 handelt es sich um ein Kunststoffmaterial.

In der Darstellung von Fig. 3 ist ein Bereich einer Grenzfläche zwischen dem ersten Werkstoff 110 und dem zweiten Werkstoff 120 zusätzlich als ein vergrößerter Ausschnitt gezeigt. In dem vergrößerten Ausschnitt sind die elektrische Schaltung 130, beispielsweise als Sensorchip ausgeführt, die Integrationsoberfläche 112 und die Verbindungselemente 114, beispielsweise als Gitterelemente oder Gitter aus dem ersten Werkstoff 110 ausgeführt, dargestellt und explizit bezeichnet. Die elektrische Schaltung 130 ist zumindest teilweise im Bereich der Verbindungselemente 114 angeordnet. Auch ist die elektrische Schaltung 130 in dem Volumen des zweiten Werkstoffs 120 eingebettet.

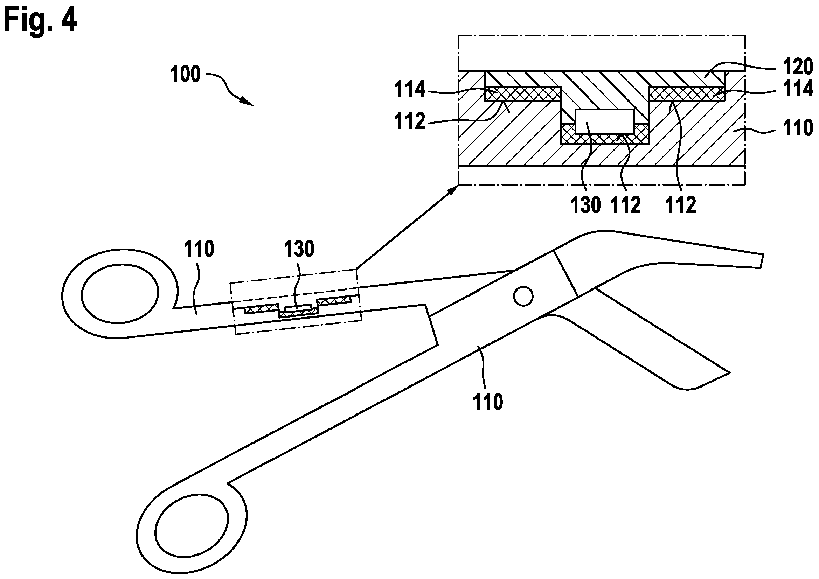

Fig. 4 zeigt eine schematische Darstellung einer Vorrichtung 100 gemäß einem Ausführungsbeispiel. Die Vorrichtung 100 in Fig. 4 entspricht oder ähnelt hierbei der Vorrichtung aus Fig. 1. Gemäß dem in Fig. 4 dargestellten Ausführungsbeispiel ist die Vorrichtung 100 als eine Schere insbesondere zur Verwendung im medizinischen Bereich ausgeführt. Dabei repräsentiert ein Korpus der Schere das Volumen des ersten Werkstoffs 110. Bei dem ersten Werkstoff 110 handelt es sich um ein Metallmaterial. Die Integrationsoberfläche 112 repräsentiert einen ausgenommenen Teilabschnitt der Schere. Genauer gesagt ist die Integrationsoberfläche 112 stufenförmig bzw. mit einem stufenförmigen Profil ausgeformt. Eine auf einem Bereich der Integrationsoberfläche 112 aufgebrachte Deckschicht repräsentiert das Volumen des zweiten Werkstoffs 120. Bei dem zweiten Werkstoff 120 handelt es sich um ein Kunststoffmaterial.

In der Darstellung von Fig. 4 ist ein Bereich der Integrationsoberfläche 112 bzw. einer Grenzfläche zwischen dem ersten Werkstoff 110 und dem zweiten Werkstoff 120 zusätzlich als ein vergrößerter Ausschnitt gezeigt. In dem vergrößerten Ausschnitt sind die elektrische Schaltung 130, beispielsweise als Sensorchip ausgeführt, die Integrationsoberfläche 112, beispielsweise stufenförmig ausgeformt bzw. mit stufenförmigen Profil, und die Verbindungselemente 114, beispielsweise als Gitterelemente oder Gitter aus dem ersten Werkstoff 110 ausgeführt, dargestellt und explizit bezeichnet. Die elektrische Schaltung 130 ist in dem die Integrationsoberfläche 112 aufweisenden, ausgenommenen Teilabschnitt des Volumens des ersten Werkstoffs 110 angeordnet und von dem Volumen des zweiten Werkstoffs 120 umschlossen.

Unter Bezugnahme auf die vorstehend beschriebenen Figuren und insbesondere auf Fig. 3 und Fig. 4 werden nachfolgend Design und Herstellung einer Vorrichtung 100 in Gestalt eines Metall-Kunststoff-Verbund-Werkzeugs mit integriertem Sensor als elektrische Schaltung 130 zusammenfassend und mit anderen Worten nochmals erläutert.

Als Verbindungselemente 114 werden metallische Gitterstrukturen in ein Design des Werkzeugs bzw. der Vorrichtung 100 integriert, welche durch additive Fertigungsverfahren, wie z. B. Elektronenstrahlschmelzen oder selektives Laserschmelzen, ausgeformt bzw. fertigungstechnisch realisiert werden. Die elektrische Schaltung 130 bzw. der Sensor (bei Bedarf in verkapselter Form) kann auf diese Gitterstrukturen bzw. Verbindungselemente 114 aufgebracht werden. Durch anschließendes Umspritzen der Gitterstrukturen bzw. Verbindungselemente 114 wird die elektrische Schaltung 130 fest in das Werkzeug bzw. die Vorrichtung 100 eingebaut. Somit kann eine vor Beschädigung schützende, dichte und damit gut sterilisierbare Integration der elektrischen Schaltung 130 erreicht werden. Zusätzlich oder alternativ zu dem einem bereits erwähnten RFID-Sensor, können z. B. auch Temperatursensoren als elektrische Schaltung 130 integriert werden, um eventuelle Sterilisationsprozesse im Bereich der Medizintechnik überwachen und dokumentieren zu können.

Somit wird eine Integration einer elektrischen Schaltung, beispielsweise eines Sensors, an verschiedenen Bereichen eines Werkzeugs bzw. einer Vorrichtung 100 ermöglicht. Unter Bezugnahme auf Fig. 3 ist anzumerken, dass die elektrische Schaltung 130 in einen Griffbereich einer als Werkzeug mit Kunststoffgriff ausgeführten Vorrichtung 100 bzw. in einen Übergangsbereich zwischen metallischem Werkzeug und Kunststoffgriff integriert ist. Unter Bezugnahme auf Fig. 4 ist anzumerken, dass die elektrische Schaltung 130 an einer beliebigen Stelle der als metallisches Werkzeug ausgeführten Vorrichtung 100 integriert ist, wobei an der Stelle die als Gitterstruktur ausgeformten Verbindungselemente 114 ausgeformt sind, wobei nach dem einbringen der elektrischen Schaltung 130 eine Füllung bzw. ein Umspritzen mit Kunststoff als zweiten Werkstoff 120 erfolgt. Als Alternative zum Umspritzen der elektrischen Schaltung 130 mit Kunststoff kann auch ein Einbetten der elektrischen Schaltung 130 in Epoxidharz zur Realisierung einer Ummantelung mit biokompatiblem Material erfolgen. Als Alternativen zur Realisierung einer Verbindung von Metall und Kunststoff als erster Werkstoff 110 und zweiter Werkstoff 120 sind auch weitere Fertigungstechnologien denkbar, wie beispielsweise Laserstrukturieren der Integrationsoberfläche 112, Strukturieren der Integrationsoberfläche 112 mittels Funkenerosion, Umformende Strukturierung der Integrationsoberfläche 112 und/oder Strukturieren der Integrationsoberfläche 112 durch Zerspanung.

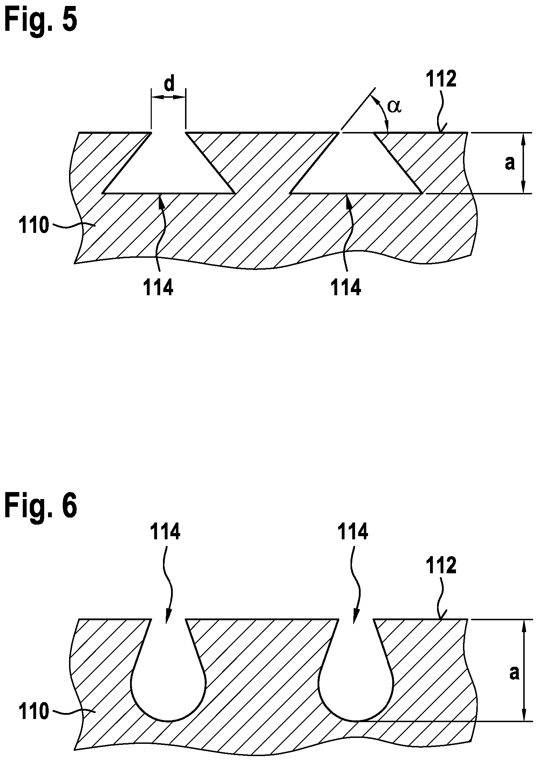

In den nachfolgend beschriebenen Figuren 5 bis 15 sind für die Vorrichtung 100 aus einer der vorstehend beschriebenen Figuren verschiedene Ausführungen von Verbindungselementen 114 gemäß Ausführungsbeispielen dargestellt, anders ausgedrückt insbesondere Varianten für eine Verbindungsstelle zwischen Metall und Kunststoff.

Fig. 5 zeigt eine schematische Darstellung von Verbindungselementen 114 gemäß einem Ausführungsbeispiel. Die Verbindungselemente 114 entsprechen oder ähneln den Verbindungselementen aus einer der vorstehend beschriebenen Figuren mit Ausnahme dessen, dass die Verbindungselemente 114 in Fig. 5 als Kavitäten mit Hinterschnitten in Schwalbenschwanzform ausgeformt sind. Gezeigt sind ein Teil des Volumens des ersten Werkstoffs 110, die Integrationsoberfläche 112 und beispielhaft lediglich zwei Verbindungselemente 114.

Die als Kavitäten mit Hinterschnitten bzw. hier einem Profil in Schwalbenschwanzform ausgeformten Verbindungselemente 114 weisen eine Öffnungsabmessung d, eine Tiefenabmessung a und einen Hinterschnittwinkel α auf. Die Öffnungsabmessung d repräsentiert eine Abmessung einer Öffnung jedes Verbindungselements 114 in der Integrationsoberfläche 112. Rein beispielhafte Werte für die Öffnungsabmessung d können zwischen 0,5 und 3 Millimetern liegen. Die Tiefenabmessung a repräsentiert eine Tiefe jedes Verbindungselements 114 relativ zu der Integrationsoberfläche 112. Rein beispielhafte Werte für die Tiefenabmessung a können zwischen 1 und 5 Millimetern liegen. Der Hinterschnittwinkel α repräsentiert einen Neigungswinkel von seitlichen Flanken jedes Verbindungselements 114 relativ zu einer Ebene der angrenzenden Integrationsoberfläche 112. Rein beispielhafte Werte für den Hinterschnittwinkel α können zwischen 45 und 70 Grad liegen.

Fig. 6 zeigt eine schematische Darstellung von Verbindungselementen 114 gemäß einem Ausführungsbeispiel. Die Verbindungselemente 114 entsprechen hierbei den Verbindungselementen aus Fig. 5 mit Ausnahme dessen, dass die Verbindungselemente 114 in Fig. 6 als Kavitäten mit Hinterschnitten in Tropfenform ausgeformt sind. Hierbei sind ebenfalls ein Teilabschnitt des Volumens des ersten Werkstoffs 110, die Integrationsoberfläche 112 und beispielhaft lediglich zwei Verbindungselemente 114 gezeigt.

Die als Kavitäten mit Hinterschnitten bzw. hier einem Profil in Tropfenform ausgeformten Verbindungselemente 114 weisen eine Tiefenabmessung a auf. Die Tiefenabmessung a repräsentiert eine Tiefe jedes Verbindungselements 114 relativ zu der Integrationsoberfläche 112. Rein beispielhafte Werte für die Tiefenabmessung a können zwischen 1 und 5 Millimetern liegen.

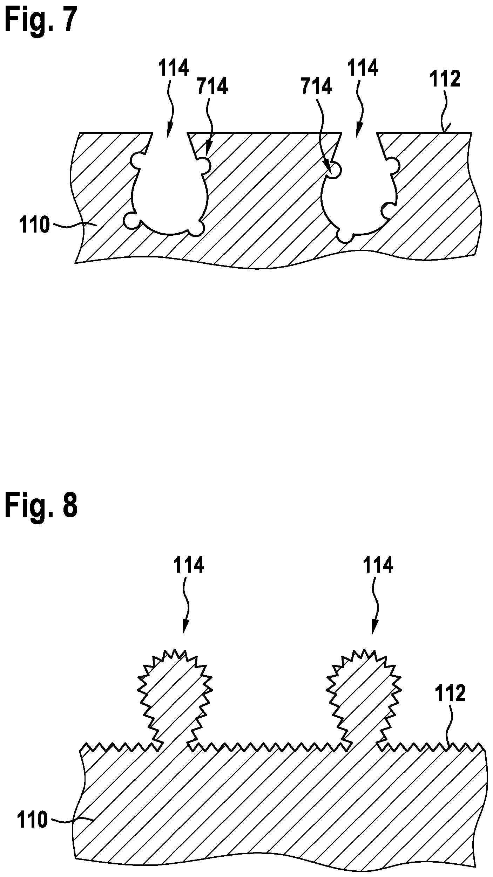

Fig. 7 zeigt eine schematische Darstellung von Verbindungselementen 114 gemäß einem Ausführungsbeispiel. Die Verbindungselemente 114 entsprechen hierbei den Verbindungselementen aus Fig. 6 mit Ausnahme dessen, dass die Verbindungselemente 114 in Fig. 7 zusätzlich Mikrokavitäten 714 aufweisen, die in einer Oberfläche der tropfenförmig ausgeformten Verbindungselemente 114 ausgeformt sind. Eine Abmessung der Mikrokavitäten 714 kann beispielsweise mindestens etwa 0,5 Millimeter betragen.

Fig. 8 zeigt eine schematische Darstellung von Verbindungselementen 114 gemäß einem Ausführungsbeispiel. Die Verbindungselemente 114 entsprechen oder ähneln den Verbindungselementen aus einer der vorstehend beschriebenen Figuren mit Ausnahme dessen, dass die Verbindungselemente 114 in Fig. 8 als tropfenförmig Ausstülpungen relativ zu der Integrationsoberfläche 112 ausgeformt sind. Ferner ist eine Rauigkeit der Integrationsoberfläche 112 und der Verbindungselemente 114 erhöht. So kann eine Fläche und damit eine Adhäsion des zweiten Werkstoffs an der Integrationsoberfläche 112 aus dem ersten Werkstoff 110 vergrößert werden. Die Rauigkeit der Integrationsoberfläche 112 und der Verbindungselemente 114 kann beispielsweise durch einen Rz-Wert von zumindest 100 Mikrometer gekennzeichnet sein.

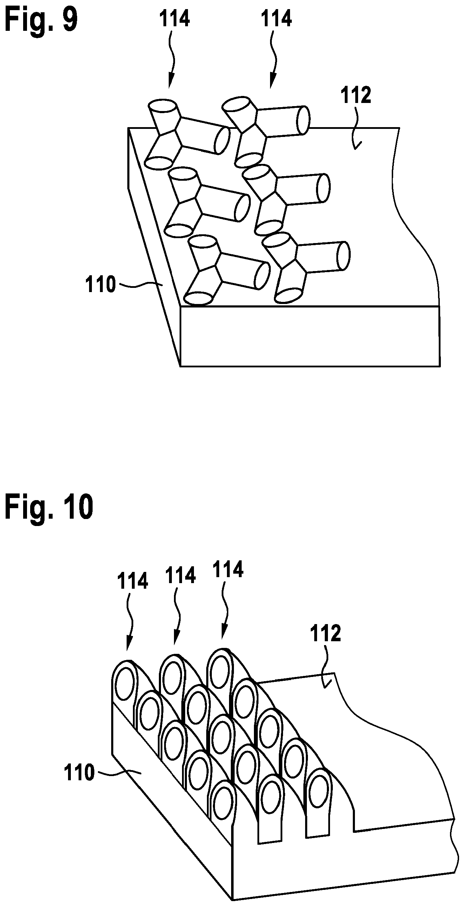

Fig. 9 zeigt eine schematische Darstellung von Verbindungselementen 114 gemäß einem Ausführungsbeispiel. Die Verbindungselemente 114 entsprechen oder ähneln den Verbindungselementen aus einer der vorstehend beschriebenen Figuren mit Ausnahme dessen, dass die Verbindungselemente 114 in Fig. 9 als Gitterstrukturen, genauer gesagt als dreidimensionale Gitterstrukturen ausgeformt sind. Gezeigt sind hierbei ein Teilabschnitt des Volumens des ersten Werkstoffs 110, die Integrationsoberfläche 112 und eine Mehrzahl von Verbindungselementen 114. Die elektrische Schaltung der Vorrichtung kann in einem Bereich der Verbindungselemente 114 eingebracht und beispielsweise mit einem Kunststoffmaterial als zweiten Werkstoff umspritzt werden.

Fig. 10 zeigt eine schematische Darstellung von Verbindungselementen 114 gemäß einem Ausführungsbeispiel. Die Verbindungselemente 114 entsprechen oder ähneln den Verbindungselementen aus einer der vorstehend beschriebenen Figuren mit Ausnahme dessen, dass die Verbindungselemente 114 in Fig. 10 als Schlaufen bzw. Verschlaufungen oder anders ausgedrückt schlaufenförmig ausgeformt sind. Gezeigt sind hierbei ein Teilabschnitt des Volumens des ersten Werkstoffs 110, die Integrationsoberfläche 112 und eine Mehrzahl von Verbindungselementen 114.

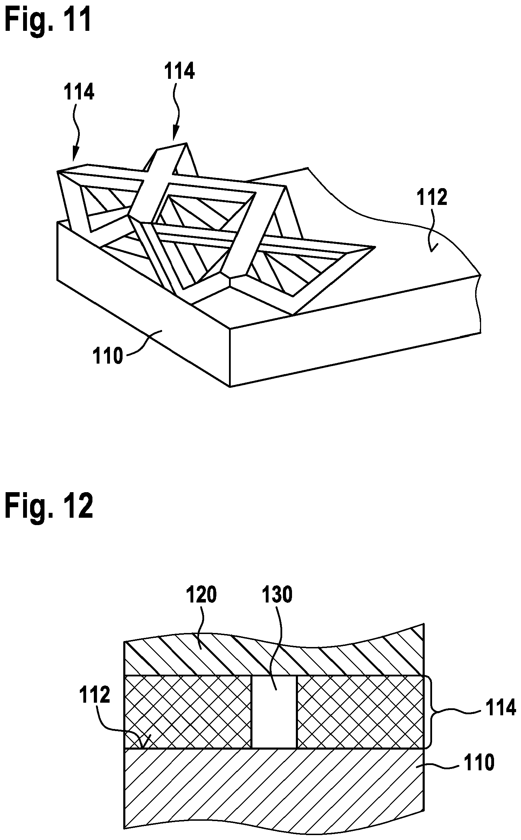

Fig. 11 zeigt eine schematische Darstellung von Verbindungselementen 114 gemäß einem Ausführungsbeispiel. Die Verbindungselemente 114 entsprechen den Verbindungselementen aus Fig. 9 mit Ausnahme dessen, dass die Verbindungselemente in Fig. 11 als anders gestaltete Gitterstrukturen ausgeformt sind. Anders ausgedrückt weisen die Gitterstrukturen in Fig. 11 eine andere Form als die Gitterstrukturen aus Fig. 9 auf.

Fig. 12 zeigt eine schematische Darstellung von Verbindungselementen 114 gemäß einem Ausführungsbeispiel. Die Verbindungselemente 114 entsprechen oder ähneln den Verbindungselementen aus einer der vorstehend beschriebenen Figuren mit Ausnahme dessen, dass die Verbindungselemente 114 in Fig. 12 als zumindest eine Antenne ausgeformt sind. Gemäß dem in Fig. 12 dargestellten Ausführungsbeispiel sind die Verbindungselemente 114 als eine Rechteckantenne ausgeformt. Gezeigt sind in Fig. 12 ein Teilabschnitt des Volumens des ersten Werkstoffs 110, die Integrationsoberfläche 112, die Verbindungselemente 114 und die elektrische Schaltung 130, welche beispielsweise als ein RFID-Chip (RFID = = Radio-Frequency Identification; Identifizierung mit Hilfe elektromagnetischer Wellen) ausgeführt ist. Ferner ist das Volumen des zweiten Werkstoffs 120 angedeutet. Die Verbindungselemente 114 und die elektrische Schaltung 130 sind durch das Volumen des zweiten Werkstoffs 120 umspritzt, umgeben bzw. eingegossen.

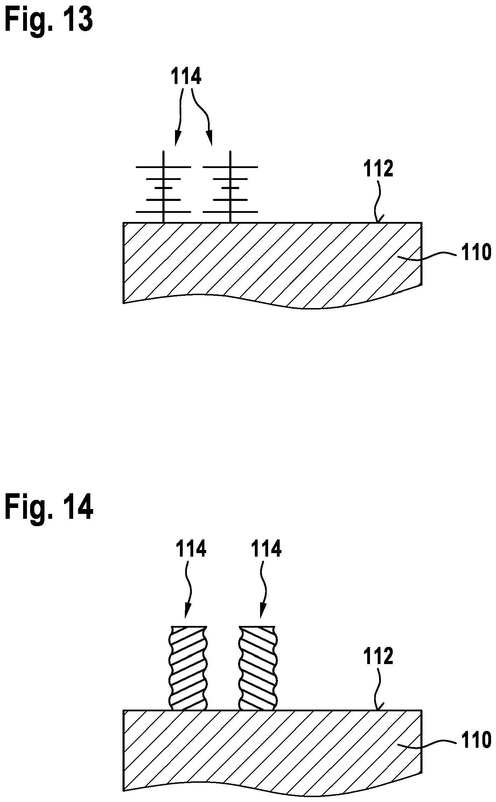

Fig. 13 zeigt eine schematische Darstellung von Verbindungselementen 114 gemäß einem Ausführungsbeispiel. Dabei entsprechen die Verbindungselemente 114 den Verbindungselementen aus Fig. 12 mit Ausnahme dessen, dass die Verbindungselemente 114 in Fig. 13 als zumindest eine Schmetterlingsantenne, hier beispielsweise zwei Schmetterlingsantennen, ausgeformt sind. Gezeigt sind in Fig. 13 ein Teilabschnitt des Volumens des ersten Werkstoffs 110, die Integrationsoberfläche 112 und beispielhaft lediglich zwei als Schmetterlingsantennen ausgeformte Verbindungselemente 114.

Fig. 14 zeigt eine schematische Darstellung von Verbindungselementen 114 gemäß einem Ausführungsbeispiel. Dabei entsprechen die Verbindungselemente 114 den Verbindungselementen aus Fig. 12 bzw. Fig. 13 mit Ausnahme dessen, dass die Verbindungselemente 114 in Fig. 14 als zumindest eine Wendelantenne, hier beispielsweise zwei Wendelantennen, ausgeformt sind. Gezeigt sind in Fig. 14 ein Teilabschnitt des Volumens des ersten Werkstoffs 110, die Integrationsoberfläche 112 und beispielhaft lediglich zwei als Wendelantennen ausgeformte Verbindungselemente 114.

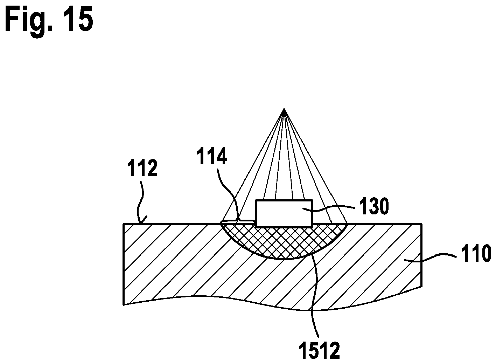

Fig. 15 zeigt eine schematische Darstellung von Verbindungselementen 114 gemäß einem Ausführungsbeispiel. Dabei entsprechen die Verbindungselemente 114 den Verbindungselementen aus Fig. 12 mit Ausnahme dessen, dass in der Integrationsoberfläche 112 ein Vertiefungsabschnitt 1512 ausgeformt ist, in dem die als zumindest eine Antenne fungierenden Verbindungselemente 115 angeordnet sind. Der Vertiefungsabschnitt 1512 ist als eine Mulde ausgeformt, insbesondere als eine Mulde mit parabolischem Vertiefungsprofil. Somit fungiert der Vertiefungsabschnitt 1512 als ein Parabolspiegel zur gebündelten Strahlabgabe. Ferner sind in Fig. 15 Teilabschnitt des Volumens des ersten Werkstoffs 110 und die als RFID-Chip ausgeführte elektrische Schaltung 130 gezeigt.

Unter Bezugnahme auf die vorstehend beschriebenen Figuren und insbesondere auf Fig. 12 bis Fig. 15 wird nachfolgend die zusätzliche Funktionalität der Verbindungselemente 114 als Antenne oder Antennen in der Verbindungszone Metall-Kunststoff erläutert.

Hierbei erfolgt eine Nutzung der gemäß Ausführungsbeispielen ohnehin vorgesehenen Verbindungselemente 114 bzw. 3D-Strukturen des Metall-Kunststoff-Werkzeugs bzw. der Vorrichtung 100 als Antenne zur Richtung von Strahlung bzw. Erhöhung einer Strahlungsreichweite von z. B. RFID-Chips als elektrischen Schaltungen 130. Fig. 12 bis Fig. 15 zeigen in diesem Zusammenhang Ausführungsbeispiele von Antennenstrukturen in der Verbindungszone Metall-Kunststoff der Vorrichtung 100 bzw. bei sensorintegrierten Werkzeugen wie der Vorrichtung 100.

Eine Formgebung und Ausrichtung der Antennen kann detailliert auf eine Sendefrequenz der elektrischen Schaltung 130 (bei RFID-Chips üblicherweise im MHz-Bereich) abgestimmt sein. Um sowohl eine stabile Verbindung zwischen den Werkstoffen 110 und 120 als auch vorteilhafte Sendeeigenschaften der Antennen zu erreichen, weist zum Beispiel die in Fig. 14 dargestellte Wendelantenne radiale Mikrokavitäten auf, welche zu einer stabilen Verbindung beim Umspritzen des Metallteiles mit Kunststoff führen sollen. Eine Verstärkungswirkung bzw. Strahlbündelung ist in ihrer Richtung abhängig von einer Ausrichtung der Antennen. Daher ist es vorteilhaft, die Antennenstrukturen mehrdimensional anzuordnen, welches z. B. mit 3D-Druck fertigungstechnisch einfach realisierbar ist.

Unter Bezugnahme auf die vorstehend beschriebenen Figuren werden Ausführungsbeispiele sowie Vorteile von Ausführungsbeispielen mit anderen Worten nochmals zusammenfassend erläutert und/oder kurz vorgestellt.

Sensorintegrierte Werkzeuge wie die Vorrichtung 100 können beispielsweise im Bereich der Medizintechnik eingesetzt werden. Hierzu kann eine als Sensor ausgeführte elektrische Schaltung 130 durch das Verfahren 200 zum Integrieren beispielsweise sterilisationssicher in die Vorrichtung integriert sein, d. h. dicht verpackt und so befestigt, dass der Sterilisationsprozess nicht einen Halt und eine Funktion des Sensors beeinträchtigt. Des Weiteren kann ein sicherer Halt der elektrischen Schaltung 130 an der Vorrichtung 100 erreicht werden, z. B. für eine eindeutige Zuordnung, eine zweifelsfreie Kontrolle eines Sterilisationsvorgangs der Vorrichtung 100 und dergleichen, was nur durch nicht ohne weiteres demontierbare Integration der elektrischen Schaltung 130 gegeben ist. Insgesamt kann beispielsweise eine Sensorintegration in Metallbauteile in Anbetracht hoher Prozesstemperaturen im Herstellungsprozess metallischer Bauteile (z. B. Gießen) vereinfacht werden, da eine Funktion der Elektronik vor Temperaturen > 100°C geschützt werden kann. Des Weiteren können Signalübertragung bzw. Kommunikation und Energieversorgung von elektrischen Schaltungen 130, welche in Metall eingebettet sind, unter Vermeidung oder zumindest Minimierung elektromagnetischer Abschirmungseffekte verbessert werden.

Gemäß einem Ausführungsbeispiel können insbesondere Sensoren in metallische Werkzeuge integriert werden und kann dabei sowohl eine Kommunikation, z. B. für ein "Tracking" bzw. Lokalisieren und Verfolgen von Werkzeugen mittels RFID, eine Nachverfolgung eines Sterilisationsprozesses etc., als auch eine Energieversorgung der Sensoren ermöglicht werden, z. B. durch induktives Laden. Der Sensor wird in den Kunststoffbereich bzw. das Volumen des zweiten Werkstoffs 120 des Werkzeugs bzw. der Vorrichtung 100 eingebracht, um eine kabellose Kommunikation und Energieversorgung des Sensors zu ermöglichen. Bei einer Einbettung eines Sensors in den Metallbereich bzw. das Volumen des ersten Werkstoffs 110 des Werkzeuges bzw. der Vorrichtung 100 wäre ein Auslesen und induktives Laden des Sensors aufgrund von Abschirmungseffekten erschwert.

Durch ein Umspritzen der Verbindungselemente 114, beispielsweise Gitterstrukturen, wird der Sensor fest in das Werkzeug Beziehung Weise die Vorrichtung 100 eingebaut. Auf diese Weise ist der Sensor so integriert, dass das Werkzeug bzw. die Vorrichtung weiterhin gut zu reinigen ist, ohne den Sensor zu beschädigen oder seinen Sitz zu beeinträchtigen. Das ist insbesondere im Bereich der Medizintechnik vorteilhaft, wenn sensorintegrierte Werkzeuge bzw. Vorrichtungen 100 sterilisiert werden sollen. Des Weiteren bietet die Kunststoffummantelung Schutz vor mechanischer Beschädigung des Sensors, z. B. Herunterfallen des Werkzeugs bzw. der Vorrichtung 100. Durch das Einbetten des Sensors in den Kunststoffbereich des Werkzeugs bzw. der Vorrichtung 100 kann eine haltbare, dauerhafte und sterilisierbare Integration des Sensors realisiert werden, welche den Sensor zusätzlich vor Beschädigung z. B. durch Herunterfallen schützt.

Durch das Einbetten des Sensors in den Kunststoffbereich des Werkzeugs bzw. der Vorrichtung 100 werden eine Kommunikation des Sensors, z. B. Auslesen eines RFID-Chips, und eine kabellose Energieversorgung, z. B. induktives Laden, ermöglicht, da der metallische Bereich bzw. das Volumen des ersten Werkstoffs 110 des Werkzeuges bzw. der Vorrichtung den Sensor in dieser Lage nicht abschirmt. Es können eine Erhöhung der Strahlungsreichweite der integrierten Sensoren bzw. elektrischen Schaltungen 130 durch Bündelung der, üblicherweise kugelförmigen, Strahlung mittels speziell geformter 3D-Strukturen bzw. -Antennen und/oder eine Erhöhung einer Reichweite in mehreren Raumrichtungen durch mehrdimensionale Ausrichtung der Strukturen bzw. Antennen ermöglicht werden. Um den Sensor bzw. die elektrische Schaltung 130 vor bei der Ummantelung auftretenden Prozesstemperaturen zu schützen, kann die elektrische Schaltung 130 verkapselt werden, z. B. mittels einer Kunststoff- oder Glaskapsel mit thermischer Schutzschicht.

Die Vorrichtung 100 und/oder das Verfahren 200 kann zur Sensorintegration in Werkzeuge allgemein eingesetzt werden. Vorteilhaft ist insbesondere auch ein Einsatz im Bereich der Medizintechnik. Es kann somit dem Bedarf begegnet werden, medizinische Werkzeuge/OP-Werkzeuge mit Sensoren auszustatten, um sie z. B. mittels RFID verfolgen zu können oder einen Sterilisationsprozess nachvollziehen bzw. verifizieren zu können. Gemäß dem Verfahren 200 in eine Vorrichtung 100 integrierte elektrische Schaltungen 130 können dazu dauerhaft und sterilisationssicher integriert werden. Es ist auch eine Anwendung zur Bestimmung eines Aufenthaltsortes von Werkzeugen bzw. Vorrichtungen 100 allgemein denkbar, beispielsweise im Bereich der Logistik und einer Werkzeugverwaltung. Ermöglicht wird dies unter anderem durch die Verbindungselemente 114, die einerseits die elektrische Schaltung 130 weit vom Metall in geringer Abschirmung halten und zudem aufgrund ihrer Form die Metall-Kunststoff-Verbindung robust und solide machen.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (200) zum Integrieren einer elektrischen Schaltung (130) in eine Vorrichtung (100), die eine Integrationsoberfläche (112) aus einem ersten Werkstoff (110) aufweist, wobei das Verfahren (200) zumindest folgende Schritte umfasst:
Bearbeiten (210) der Integrationsoberfläche (112), um Verbindungselemente (114) zum Erhöhen einer Adhäsion eines zweiten Werkstoffs (120) an der Integrationsoberfläche (112) auszuformen, wobei sich der zweite Werkstoff (120) von dem ersten Werkstoff (110) unterscheidet;
Anordnen (220) der elektrischen Schaltung (130) benachbart zu der bearbeiteten Integrationsoberfläche (112); und
Aufbringen (230) eines Volumens des zweiten Werkstoffs (120) zumindest über die Integrationsoberfläche (112), um die elektrische Schaltung (130) fluiddicht einzuhausen, **dadurch gekennzeichnet,**
**dass** bei dem Schritt (210) des Bearbeitens die Verbindungselemente (114) mittels eines additiven Fertigungsprozesses ausgeformt werden.

2. Verfahren (200) gemäß Anspruch 1, bei dem im Schritt (210) des Bearbeitens Gitterelemente, Ausnehmungen, Schlaufen, Vorsprungsabschnitte, Winkelelemente, Kavitäten mit Hinterschnitten und/oder Kavitäten zur Erhöhung eines Rauigkeitswertes als Verbindungselemente (114) ausgeformt werden.

3. Verfahren (200) gemäß einem der vorangegangenen Ansprüche, bei dem im Schritt (210) des Bearbeitens die Verbindungselemente (114) zusätzlich mittels Laserstrukturierens, Funkenerodierens, Umformens und/oder Zerspanens ausgeformt werden.

4. Verfahren (200) gemäß einem der vorangegangenen Ansprüche, bei dem im Schritt (210) des Bearbeitens zumindest ein Teil der Verbindungselemente (114) als zumindest eine Antenne für Signale zu und/oder von der elektrischen Schaltung (130) ausgeformt werden.

5. Verfahren (200) gemäß Anspruch 4, bei dem die zumindest eine Antenne als eine Wendelantenne, als eine Wendelantenne mit radialen Mikrokavitäten, als eine Rechteckantenne und/oder als eine Schmetterlingsantenne ausgeformt wird.

6. Verfahren (200) gemäß einem der Ansprüche 4 bis 5, bei dem eine Mehrzahl von Antennen ausgeformt wird, die in unterschiedlichen Dimensionen ausgerichtet sind.

7. Verfahren (200) gemäß einem der Ansprüche 4 bis 6, bei dem im Schritt (210) des Bearbeitens zumindest ein Vertiefungsabschnitt (1512) in der Integrationsoberfläche (112) ausgeformt wird, wobei der zumindest eine Vertiefungsabschnitt (1512) ein parabolisches Vertiefungsprofil aufweist.

8. Verfahren (200) gemäß einem der vorangegangenen Ansprüche, bei dem im Schritt (220) des Anordnens eine elektrische Schaltung (130) angeordnet wird, die zumindest eine Erfassungseinrichtung zum Erfassen einer physikalischen Größe und/oder eine Signalübertragungseinrichtung zum Übertragen von Signalen zu und/oder von der elektrischen Schaltung (130) aufweist.

9. Verfahren (200) gemäß einem der vorangegangenen Ansprüche, bei dem im Schritt (220) des Anordnens die elektrische Schaltung (130) mit einem Kapselungsmaterial verkapselt wird und/oder mit einer Wärmeschutzschicht versehen wird.

10. Vorrichtung (100), die zumindest folgende Merkmale aufweist:
eine Integrationsoberfläche (112) aus einem ersten Werkstoff (110), wobei an der Integrationsoberfläche (112) Verbindungselemente (114) zum Erhöhen einer Adhäsion eines zweiten Werkstoffs (120) an der Integrationsoberfläche (112) ausgeformt sind, wobei sich der zweite Werkstoff (120) von dem ersten Werkstoff (110) unterscheidet;
eine benachbart zu der bearbeiteten Integrationsoberfläche (112) angeordnete elektrische Schaltung (130); und
ein zumindest über die Integrationsoberfläche (112) aufgebrachtes Volumen des zweiten Werkstoffs (120) zum fluiddichten Einhausen der elektrischen Schaltung (130) **dadurch gekennzeichnet, dass** die Verbindungselemente (114) mittels eines additiven Fertigungsprozesses ausgeformt werden.

11. Vorrichtung (100) gemäß Anspruch 10, die als ein medizinisches Gerät ausgeformt ist, wobei die Integrationsoberfläche (112) an einem Griff des medizinischen Geräts ausgeformt ist.

## Claims

1. Method (200) for integrating an electrical circuit (130) in a device (100) having an integration surface (112) composed of a first material (110), wherein the method (200) comprises at least the following steps:
processing (210) the integration surface (112) in order to shape connection elements (114) for increasing an adhesion of a second material (120) to the integration surface (112), wherein the second material (120) differs from the first material (110);
arranging (220) the electrical circuit (130) adjacent to the processed integration surface (112); and
applying (230) a volume of the second material (120) at least over the integration surface (112) in order to enclose the electrical circuit (130) in a fluid-tight manner, **characterized**
**in that**, in step (210) of processing, the connection elements (114) are shaped by means of an additive manufacturing process.

2. Method (200) according to Claim 1, wherein, in step (210) of processing, grid elements, recesses, loops, projection sections, angular elements, cavities having undercuts and/or cavities for increasing a roughness value are shaped as connection elements (114).

3. Method (200) according to either of the preceding claims, wherein, in step (210) of processing, the connection elements (114) are additionally shaped by means of laser structuring, spark erosion, forming and/or machining.

4. Method (200) according to any of the preceding claims, wherein, in step (210) of processing, at least one portion of the connection elements (114) are shaped as at least one antenna for signals to and/or from the electrical circuit (130).

5. Method (200) according to Claim 4, wherein the at least one antenna is shaped as a helical antenna, as a helical antenna having radial microcavities, as a rectangular antenna and/or as a butterfly antenna.

6. Method (200) according to either of Claims 4 to 5, wherein a plurality of antennas aligned in different dimensions are shaped.

7. Method (200) according to any of Claims 4 to 6, wherein, in step (210) of processing, at least one depression section (1512) is shaped in the integration surface (112), wherein the at least one depression section (1512) has a parabolic depression profile.

8. Method (200) according to any of the preceding claims, wherein, in step (220) of arranging, an electrical circuit (130) is arranged which has at least one detection unit for detecting a physical variable and/or a signal transmission unit for transmitting signals to and/or from the electrical circuit (130).

9. Method (200) according to any of the preceding claims, wherein, in step (220) of arranging, the electrical circuit (130) is encapsulated with an encapsulation material and/or is provided with a thermal protection layer.

10. Device (100) having at least the following features:
an integration surface (112) composed of a first material (110), wherein connection elements (114) for increasing an adhesion of a second material (120) to the integration surface (112) are shaped on the integration surface (112), wherein the second material (120) differs from the first material (110);
an electrical circuit (130) arranged adjacent to the processed integration surface (112); and
a volume of the second material (120) applied at least over the integration surface (112) for enclosing the electrical circuit (130) in a fluid-tight manner, **characterized in that** the connection elements (114) are shaped by means of an additive manufacturing process.

11. Device (100) according to Claim 10, which is shaped as a medical device, wherein the integration surface (112) is shaped on a handle of the medical device.

## Revendications

1. Procédé (200) permettant d'intégrer un circuit électrique (130) dans un dispositif (100) qui présente une surface d'intégration (112) composée d'un premier matériau (110), le procédé (200) comprenant au moins les étapes suivantes consistant à :
traiter (210) la surface d'intégration (112) pour configurer des éléments de liaison (114) afin d'augmenter une adhésion d'un deuxième matériau (120) à la surface d'intégration (112), le deuxième matériau (120) étant différent du premier matériau (110) ;
disposer (220) le circuit électrique (130) de manière adjacente à la surface d'intégration (112) à traiter ; et
appliquer (230) un volume du deuxième matériau (120) au moins sur la surface d'intégration (112) afin d'enrober le circuit électrique (130) de manière étanche aux fluides, **caractérisé**
**en ce qu'**à l'étape (210) de traitement, les éléments de liaison (114) sont configurés au moyen d'un processus de fabrication additif.

2. Procédé (200) selon la revendication 1, dans lequel à l'étape (210) de traitement, des éléments de grille, des évidements, des boucles, des parties en saillie, des éléments d'angle, des cavités à contre-dépouilles et/ou des cavités pour augmenter un coefficient de rugosité sont configurés comme éléments de liaison (114).

3. Procédé (200) selon l'une quelconque des revendications précédentes, dans lequel à l'étape (210) de traitement, les éléments de liaison (114) sont en outre configurés par structuration par laser, électroérosion, transformation et/ou enlèvement de copeaux.

4. Procédé (200) selon l'une quelconque des revendications précédentes, dans lequel, à l'étape (210) de traitement, au moins une partie des éléments de liaison (114) est configurée comme au moins une antenne pour des signaux à destination et/ou en provenance du circuit électrique (130).

5. Procédé (200) selon la revendication 4, dans lequel ladite au moins une antenne est configurée comme une antenne hélicoïdale, comme une antenne hélicoïdale à microcavités radiales, comme une antenne rectangulaire et/ou comme une antenne biconique.

6. Procédé (200) selon l'une quelconque des revendications 4 à 5, dans lequel une pluralité d'antennes est configurée qui sont orientées selon différentes dimensions.

7. Procédé (200) selon l'une quelconque des revendications 4 à 6, dans lequel à l'étape (210) de traitement, au moins une partie en creux (1512) est configurée dans la surface d'intégration (112), dans lequel ladite au moins une partie en creux (1512) présente un profil de creux parabolique.

8. Procédé (200) selon l'une quelconque des revendications précédentes, dans lequel à l'étape (220) de disposition, un circuit électrique (130) est disposé qui présente au moins un dispositif de détection pour détecter une grandeur physique et/ou un dispositif de transmission de signal pour transmettre des signaux à destination et/ou en provenance du circuit électrique (130) .

9. Procédé (200) selon l'une quelconque des revendications précédentes, dans lequel à l'étape (220) de disposition, le circuit électrique (130) est encapsulé avec un matériau d'encapsulation et/ou est muni d'une couche de protection thermique.

10. Dispositif (100), présentant au moins les particularités suivantes :
une surface d'intégration (112) composée d'un premier matériau (110), dans lequel des éléments de liaison (114) pour augmenter une adhésion d'un deuxième matériau (120) à la surface d'intégration (112) sont configurés au niveau de la surface d'intégration, le deuxième matériau (120) étant différent du premier matériau (110) ;
un circuit électrique (130) disposé de manière adjacente à la surface d'intégration (112) à traiter ; et
un volume du deuxième matériau (120) appliqué au moins sur la surface d'intégration pour enrober le circuit électrique (130) de manière étanche aux fluides, **caractérisé en ce que** les éléments de liaison (114) sont configurés au moyen d'un processus de fabrication additif.

11. Dispositif (100) selon la revendication 10 qui est configuré comme un appareil médical, dans lequel la surface d'intégration (112) est configurée au niveau d'une poignée de l'appareil médical.
